(19) Europäisches Patentamt
European Patent Office
Office européen des brevets

(11) Publication number: 0 322 153 A2

(12) # EUROPEAN PATENT APPLICATION

(21) Application number: 88311857.2

(22) Date of filing: 15.12.88

(51) Int. Cl.[4]: C07D 417/06 , C07D 409/06 , C07D 471/04 , C07D 411/06 , C07D 407/06 , C07D 403/06 , A61K 31/50

(30) Priority: 21.12.87 US 136179

(43) Date of publication of application: 28.06.89 Bulletin 89/26

(84) Designated Contracting States: AT BE CH DE ES FR GB GR IT LI LU NL SE

(71) Applicant: PFIZER INC.
235 East 42nd Street
New York, N.Y. 10017(US)

(72) Inventor: Larson, Eric Robert
88 Granite Road
Guilford Connecticut(US)
Inventor: Mylari, Banavara Lakshmana
6, Quinley Way
Waterford Connecticut(US)

(74) Representative: Wood, David John et al
Pfizer Limited Ramsgate Road
Sandwich Kent CT13 9NJ(GB)

(54) **Heterocyclic oxophtalazinyl acetic acids.**

(57) A heterocyclic oxophthalazinyl acetic acid having aldose reductase inhibitory activity of the formula

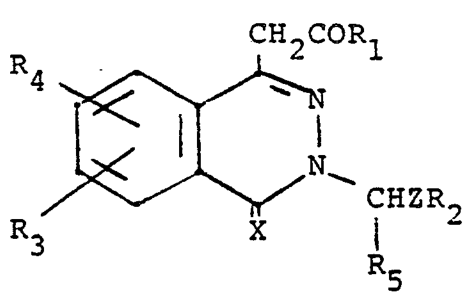

I

wherein X is oxygen or sulfur, Z is a covalent bond, O, S, NH or $CH_2$ or $CHR_5Z$ is vinylene; $R_1$ is hydroxy, or a prodrug group; $R_2$ is a heterocyclic group, $R_3$ and $R_4$ are hydrogen or the same or a different substituent, and $R_5$ is hydrogen, methyl or trifluoromethyl. The pharmaceutically acceptable acid addition salts of the above compounds wherein $R_1$ is di($C_1$-$C_4$)alkylamino or ($C_1$-$C_4$)alkoxy substituted by N-morpholino or di($C_1$-$C_4$)alkylamino and the pharmaceutically active base addition salts of the above compounds wherein $R_1$ is hydroxy are also aldose reductase inhibitors.

EP 0 322 153 A2

# HETEROCYCLIC OXOPHTHALAZINYL ACETIC ACIDS

This invention relates to novel heterocyclic oxophthalazinyl acetic acids useful in the treatment of certain chronic complications arising from diabetes mellitus, such as diabetic cataracts, retinopathy and neuropathy, and to pharmaceutical compositions containing such compounds.

The present invention is a further development of European Patent Publication No. 222576 directed to aldose reductase inhibiting oxophthalazinyl acetic acids containing at least one heterocyclic group.

According to the invention, compounds are provided having the formula

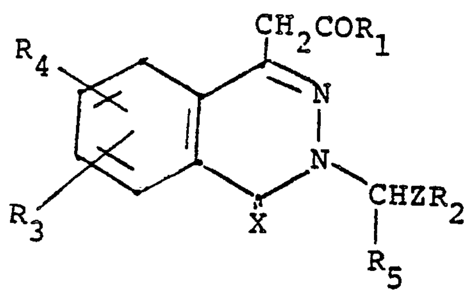

I

wherein

X is oxygen or sulfur;

Z is a covalent bond, O, S, NH or $CH_2$, or $CHR_5Z$ is vinylene;

$R_1$ is hydroxy, or a prodrug group;

$R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said heterocyclic 5-membered ring substituted by one or two phenyl, or condensed with benzo, said phenyl or benzo substituted by one of cyano, nitro, perfluoroethyl, trifluoroacetyl, or $(C_1\text{-}C_4)$alkanoyl, one or two of hydroxy or trifluoromethoxy, or one or two trifluoromethyl with one hydroxy or one $(C_1\text{-}C_4)$alkoxy, or three fluoro;

said benzo-condensed heterocyclic 5-membered ring optionally substituted in the heterocyclic 5-membered ring by one of fluoro, chloro, bromo, methoxy, or trifluoromethyl;

imidazolopyridine substituted by one of trifluoromethyl, trifluoromethylthio, bromo, or $(C_1\text{-}C_4)$alkoxy, or two of fluoro or chloro;

triazolopyridine optionally substituted by one of trifluoromethyl, trifluoromethylthio, bromo, or $(C_1\text{-}C_4)$alkoxy, or two of fluoro or chloro;

thienothiophene or thienofuran optionally substituted by one of fluoro, chloro or trifluoromethyl;

thienotriazole optionally substituted by one of chloro or trifluoromethyl;

thienopyridyl; or thienoisothiazole;

$R_3$ and $R_4$ are the same or different and are hydrogen, fluoro, chloro, bromo, trifluoromethyl, $(C_1\text{-}C_4)$alkyl, $(C_1\text{-}C_4)$alkoxy, $(C_1\text{-}C_4)$alkylthio, $(C_1\text{-}C_4)$alkylsulfinyl, $(C_1\text{-}C_4)$alkylsulfonyl, or nitro, or $R_3$ and $R_4$ taken together are $(C_1\text{-}C_4)$alkylenedioxy; and

$R_5$ is hydrogen, methyl or trifluoromethyl; or

a pharmaceutically acceptable base addition salt of a compound of formula I wherein $R_1$ is hydroxy, or an acid addition salt of a compound of formula I wherein $R_1$ is di$(C_1\text{-}C_4)$alkylamino or $(C_1\text{-}C_4)$alkoxy substituted by N-morpholino or di$(C_1\text{-}C_4)$alkylamino;

with the proviso that when $CHR_5Z$ is vinylene, $R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said heterocyclic 5-membered ring substituted by one or two fluoro, chloro, $(C_1\text{-}C_4)$alkyl or phenyl, or condensed with benzo, or substituted by one of pyridyl, furyl or thienyl, said phenyl or benzo optionally substituted by one of iodo, cyano, nitro, perfluoroethyl, trifluoroacetyl, or $(C_1\text{-}C_4)$alkanoyl, one or two of fluoro, chloro, bromo, hydroxy, $(C_1\text{-}C_4)$alkyl, $(C_1\text{-}C_4$alkoxy, $(C_1\text{-}C_4)$alkylthio, trifluoromethoxy, trifluoromethylthio, $(C_1\text{-}C_4)$alkylsulfinyl, $(C_1\text{-}C_4)$alkylsulfonyl, or trifluoromethyl, or two fluoro or two trifluoromethyl with one hydroxy or one $(C_1\text{-}C_4)$alkoxy, or three fluoro; said pyridyl, furyl or thienyl optionally substituted in the 3-position by fluoro, chloro, bromo, $(C_1\text{-}C_4)$alkyl or $(C_1\text{-}C_4)$alkoxy;

a heterocyclic 6-membered ring having one to three nitrogen atoms, or one or two nitrogen atoms and one oxygen or sulfur, said heterocyclic 6-membered ring substituted by one or two ($C_1$-$C_4$)alkyl or phenyl, or condensed with benzo, or substitued by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, ($C_1$-$C_4$)alkyl or ($C_1$-$C_4$)alkoxy;
said benzo-condensed heterocyclic 5-membered or 6-membered rings optionally substituted in the heterocyclic 5-membered or 6-membered ring by one of fluoro, chloro, bromo, methoxy, or trifluoromethyl;
oxazole or thiazole condensed with a 6-membered aromatic group containing one or two nitrogen atoms, with thiophene or with furane, each optionally substituted by one of fluoro, chloro, bromo, trifluoromethyl, methylthio or methylsulfinyl;
imidazolopyridine or triazolopyridine optionally substituted by one of trifluoromethyl, trifluoromethylthio, bromo, or ($C_1$-$C_4$)alkoxy, or two of fluoro or chloro;
thienothiophene or thienofuran optionally substituted by one of fluoro, chloro or trifluoromethyl;
thienotriazole optionally substituted by one of chloro or trifluoromethyl;
naphthothiazole, naphthoxazole; thienopyridyl; or thienoisothiazole.

More specific compounds of the invention are those of formula I wherein X is oxygen and those wherein $R_2$ is substituted benzothiazolyl, benzoxazolyl, isoquinolyl, benzothiophen-yl, benzofuran-yl or benzimidazolyl, or oxadiazolyl or indolyl. Other more specific compounds are those wherein $R_5$ is trifluoromethyl, X is oxygen, Z is a covalent bond or $CH_2$, $R_1$ is hydroxy, and $R_3$ and $R_4$ are hydrogen.

Preferred compounds of the invention are those of formula I wherein X is oxygen, Z is a covalent bond, and $R_1$ is hydroxy, those wherein $R_2$ is substituted benzothiazol-2-yl, benzothiazol-5-yl, benzoisothiazol-3-yl, benzoxazol-2-yl, benzothiophen-2-yl, benzofuran-2-yl, thieno[2,3-b]pyridine-2-yl, imidazo[1,5-a]pyridine-2-yl, indol-2-yl, 1,2,4-oxadiazol-3-yl, 1,2,4-oxadiazol-5-yl, isothiazol-5-yl, isothiazol-4-yl, 1,3,4-oxadiazol-5-yl, 1,2,5-thiadiazol-3-yl, oxazol-2-yl, thiazol-2-yl, or thiazol-4-yl, and those wherein $R_3$, $R_4$ and $R_5$ are hydrogen.

Other preferred compound are those wherein the methylene bridge connecting the oxophthalazinyl group with $R_2$ is located alpha with respect to a nitrogen atom in $R_2$, e.g. wherein $R_2$ is benzoxazol-2-yl or 1,2,4-oxadiazol-3-yl mentioned above.

Other more specific compounds of the invention are those wherein X is oxygen, Z is a covalent bond, $R_1$ is hydroxy, $R_2$ is 4,5,6 or 7 benzo-substituted benzothiazolyl, benzoxazolyl, benzimidazolyl, benzothiophenyl, benzofuranyl, or indolyl, or $R_2$ is 2-benzothiazolyl substituted in the benzo by one trifluoroacetyl, or one or two of hydroxy, or trifluoromethoxy, or two fluoro or two trifluoromethyl with one methoxy, or three fluoro; and those wherein $R_3$ is hydrogen, 5-fluoro, 5-chloro, 5-bromo or 5-methyl, and $R_4$ is hydrogen, 6- or 7- substituted chloro, bromo, methyl, isopropyl, methoxy, nitro or trifluoromethyl; or $R_3$ and $R_4$ is 4,5-difluoro; and those wherein $R_2$ is substituted benzothiazol-2-yl, or triazolopyridin-2-yl, and $R_3$ and $R_4$ are each chloro.

Further more specific compounds are those wherein X is oxygen, Z is $CH_2$, $R_1$ is hydroxy, $R_2$ is substituted benzothiazol-2-yl, benzothiazol-5-yl, benzoisothiazol-3-yl, benzoxazol-2-yl, 1,2,4,- oxadiazol-3-yl, 1,2,4-oxadiazol-5-yl, isothiazol-5-yl, isothiazol-4-yl, 1,3,4-oxadiazol-5-yl, 1,2,5-thiadiazol-3-yl, oxazol-2-yl, thiazol-2-yl, or thiazol-4-yl, and $R_3$, $R_4$ and $R_5$ are hydrogen.

Specific preferred compounds of formula I are 3-(6-hydroxy-2-benzothiazolylmethyl)-4-oxo-3H-phthalazin-1-yl-acetic acid, and 3-(5-trifluoromethyl-6-hydroxy-2-benzothiazolylmethyl)-4-oxo-3H-phthalazin-1-ylacetic acid.

The present invention also relates to a pharmaceutical composition for inhibition of aldose reductase activity comprising a compound of formula I in an amount effective in the inhibition of aldose reductase activity, in admixture with a pharmaceutically acceptable carrier. Specific and preferred compositions contain the specific and preferred compounds of formula I as described above.

The invention further comprises a method of treating a diabetic host such as an animal or a human for diabetes-associated complications which comprises administering to the host an effective amount of a compound of formula I.

The term "($C_1$-$C_4$) alkyl" whenever used in the definitions of $R_1$ to $R_4$ denotes saturated monovalent straight or branched aliphatic hydrocarbon radicals having one to four carbon atoms, such as methyl, ethyl, propyl, butyl, t-butyl etc.

The term "prodrug" denotes a group that is converted in vivo into the active compound of formula I wherein $R_1$ is hydroxy. Such groups are generally known in the art and include ester forming groups, to form an ester prodrug, such as benzyloxy, di($C_1$-$C_4$)alkylamino ethyloxy, acetoxymethyl, pivaloyloxymethyl, phthalidoyl, ethoxycarbonyloxyethyl, 5-methyl-2-oxo-1,3-dioxol-4-yl methyl, and ($C_1$-$C_4$)alkoxy optionally substituted by N-morpholino and amide-forming groups such as di($C_1$-$C_4$)alkylamino.

The heterocyclic 5-membered ring having one to three nitrogen atoms, one of which may be replaced by oxygen or sulfur includes imidazolyl, oxazolyl, thiazolyl, pyrazolyl, oxadiazolyl, thiadiazolyl, and triazolyl.

The heterocyclic 6-membered ring having one to three nitrogen atoms, or one or two nitrogen atoms and one oxygen or sulfur includes triazinyl, pyrimidyl, pyridazinyl, oxazinyl and triazinyl.

The heterocyclic ring when condensed with benzo is attached at two neighboring carbon atoms with benzo to form a phenyl group. Such benzoheterocyclic ring may be attached to Z either through the heterocyclic group or through the benzo group of the benzoheterocyclic ring. The preparation of those compounds wherein Z is attached to the benzo group is illustrated in Reaction Scheme B below. Specific examples wherein said heterocyclic ring is condensed with a benzo include benzoxazolyl, quinazolin-2-yl, 2-benzimidazolyl, quinazolin-4-yl and benzothiazolyl.

The compounds of the invention are prepared as outlined below with reference to Reaction Scheme A.

Phthalic anhydride and its derivatives of formula II are either commercially available or may be prepared according to standard procedures. The compounds of formulae III and IV wherein R′ is ethyl or methyl may be prepared as described in the above European patent publication.

The compounds of formula V are formed on reacting compounds (IV) wherein R′ is hydrogen, methyl or ethyl, with

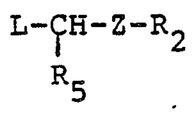

wherein L is a leaving group capable of forming compound LH on reaction of said two reagents. L is for example chloro, bromo, or $OSO_2R_6$, wherein $R_6$ is $(C_1\text{-}C_4)$alkyl, trifluoromethyl, phenyl or phenyl substituted by methyl, chloro, bromo or nitro.

REACTION SCHEME A

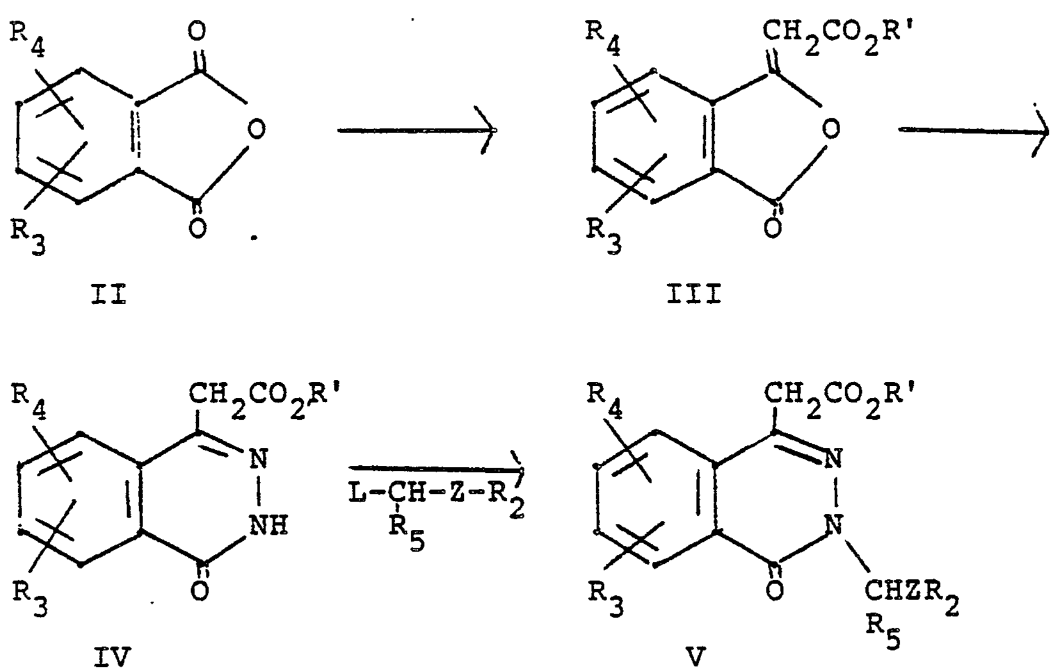

II III

IV V

When reacting compounds (IV) wherein R′ is methyl or ethyl, the process is generally carried out in a polar solvent such as an alkanol having 1 to 4 carbon atoms, e.g. methanol or ethanol, dioxan, dimethylformamide, or dimethylsulfoxide, in the presence of a base. Suitable bases are alkali metal hydride or alkoxide

of 1 to 4 carbon atoms, such as sodium or potassium hydride, methoxide or ethoxide. When a hydride is used, a non-aqueous solvent such as dimethylformamide is required. When reacting compounds (IV) wherein $R'$ is hydrogen, obtained on hydrolysis of compounds (IV) wherein $R'$ is methyl or ethyl, it is necessary for at least two molar equivalents of the base to be present, since the first molar equivalent reacts with the carboxylic acid radical of such a compound. In addition, when reacting such compounds, it is preferable to use a hydroxylic solvent to minimize production of a corresponding ester.

The reaction to form compounds (V) wherein $R_5$ is methyl or trifluoromethyl is preferably performed with compounds of formula

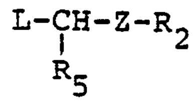

wherein $R_5$ is methyl or trifluoromethyl, and L is $OSO_2R_6$ wherein $R_6$ is as defined above. This reaction is generally conducted in an inert atmosphere such as nitrogen in an aprotic polar solvent such as dimethylformamide at temperatures of 20 to 50° C.

The reaction to form compound (V) may be at room temperature, or at higher temperatures to accelerate the process.

The compounds of formula V wherein $R'$ is methyl or ethyl may be hydrolyzed to obtain compounds of formula I wherein $R_1$ is hydrogen. The hydrolysis proceeds at conventional temperatures and in the presence of acid or base such as a mineral acid, for example hydrochloric acid, or an alkali metal hydroxide or carbonate such as sodium or potassium hydroxide or carbonate. The reaction is carried out in the presence of water and a solvent, for example an alkanol of 1 to 4 carbon atoms such as methanol, or dioxane.

The compounds of formula I wherein $R_1$ is hydroxyl may be esterified by conventional methods such as reaction of the corresponding acid chloride, bromide or anhydride with $R_1H$ to obtain compounds (I) wherein $R_1$ is an ester prodrug group. Alternatively, the compounds of formula I in which $R_1$ is an ester prodrug group may be prepared by alkylating a solution of the sodium salt of a compound (I) wherein $R_1$ is hydroxy. The alkylating agent may be a chloride. For instance, when $R_1$ is benzyloxy, acetoxymethyl, or pivaloyloxymethyl, then the alkylating agent is benzylchloride, chloromethylacetate or chloromethylpivalate, respectively. The above sodium salt is generally prepared <u>in situ</u> by reacting a compound (I) wherein $R_1$ is hydroxy with a sodium salt forming compound such as sodium bicarbonate, sodium hydride or sodium t-butylammonium sulfate in a non-aqueous solvent such as dimethylformamide or methylpyrrolidone.

When $R_1$ in compounds of formula (I) is an amide prodrug group such as di($C_1$-$C_4$)alkylamino, a compound (I) wherein $R_1$ is ($C_1$-$C_4$)alkoxy is converted to the corresponding amide by reaction with an amine, e.g. di($C_1$-$C_4$)alkylamine.

The compounds of formula I wherein X is sulfur are prepared by thiating the corresponding compounds (I) wherein X is oxygen by known procedures, for example by reaction with phosphorus pentasulphide.

An alternative method for forming the triazolopyridyl- or benzothiazolyl- substituted compounds of formula I is by reacting a compound of the formula

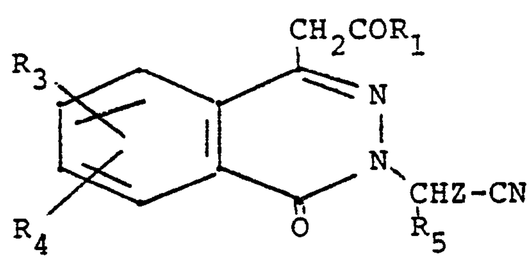

wherein $R_1$ is hydroxy or $C_1$-$C_4$ alkoxy, $R_3$, $R_4$ and $R_5$ are as defined above for the compounds of formula I, and Z is a covalent bond or $CH_2$, with an optionally substituted N-aminopyridinium mesylate or with an acid addition salt of a substituted 2-amino-thiophenol, respectively.

Reaction Scheme B exemplifies the preparation of 3-(benzothiazole-5-ylmethyl)-4-oxo-phthalazin-1-yl-acetic acid which is a compound according to the invention wherein $R_2$ is a benzo-heterocyclic ring with the benzo attached to the $-CHR_5Z-$ bridge in the final compound. Other such compounds wherein $R_2$ is a

benzo-heterocyclic ring with the benzo attached to the Z in the final compound may be made by a similar method. In Scheme B, 3-methylbenzothiazole is reacted with a brominating agent such as N-bromosuccinimide to form 5-bromomethylbenzothiazole which is then reacted with 4-oxo-3H-phthalazin-1-yl acetate to form ethyl 3-(5-methylbenzothiazolyl)-4-oxo-3H-phthalazin-1-yl acetate under conditions as outlined above with reference to Reaction Scheme A for the conversion of compounds (IV) to compounds (V).

REACTION SCHEME B

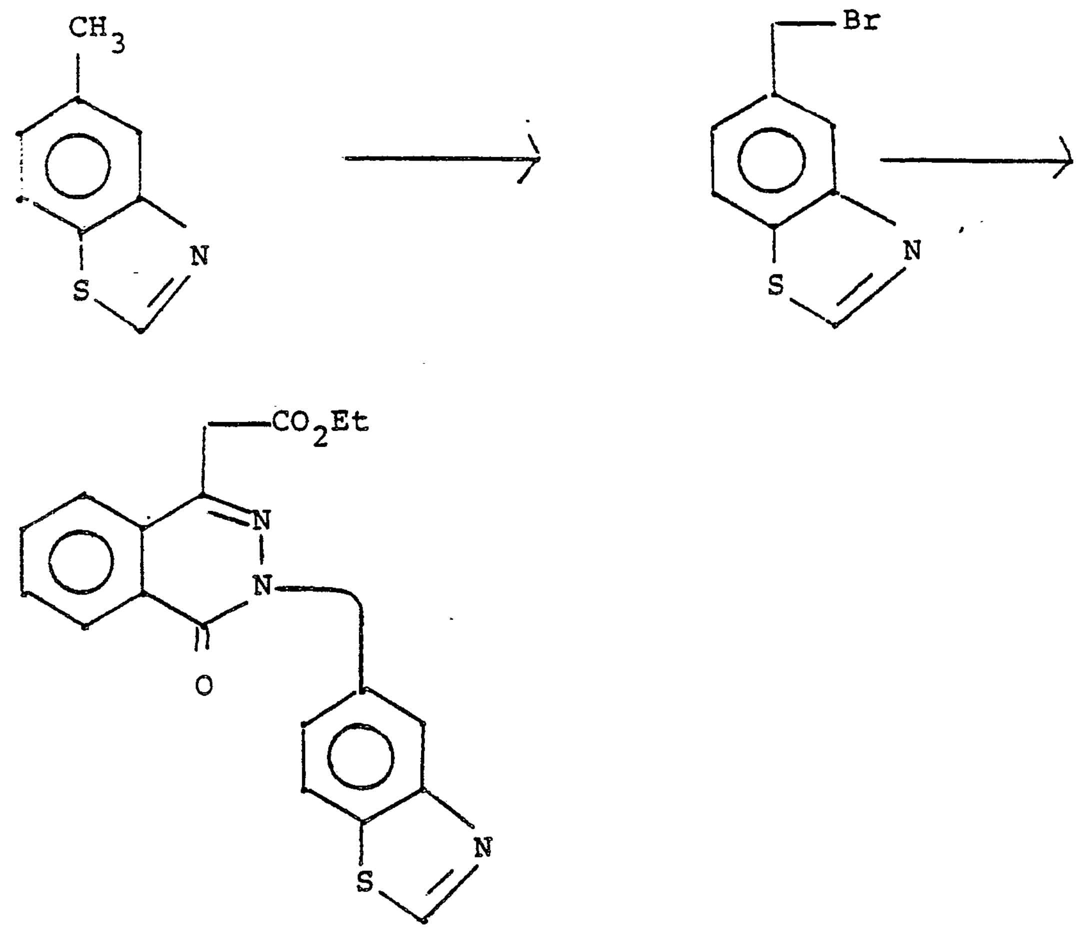

The pharmaceutically acceptable base addition salts of compounds (I) wherein $R_1$ is hydroxy may be formed with pharmaceutically acceptable cations by conventional methods. Thus, these salts may be readily prepared by treating the compound of formula I with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired metal and the solution subsequently evaporated to dryness. Suitable pharmaceutically acceptable cations for this purpose include, but are not limited to, alkali metal cations such as potassium and sodium, ammonium or water-soluble amine addition salts such as N-methylglucamine-(meglumine), the lower alkanolammonium and other base salts with organic amines which are pharmaceutically acceptable, and alkaline earth metal cations such as calcium and magnesium. In general, the sodium and N-methylglucamine salts are preferred.

The pharmaceutically acceptable acid addition salts of the compounds of formula I are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are acetic, lactic, succinic, maleic,

tartaric, citric, gluconic, ascorbic, benzoic, cinnamic, fumaric, sulfuric, phosphoric, hydroxhloric, hydrobromic, hydroiodic, sulfamic, sulfonic such as methanesulfonic, benzensulfonic, and related acids. Preferably, the acid is phosphoric acid.

The novel compounds of formula I and the pharmaceutically acceptable salts thereof are useful as inhibitors of the enzyme aldose reductase in the treatment of chronic complications of diabetes, such as diabetic cataracts, retinopathy and neuropathy. As used in the claims and specification hereof, treatment is meant to include both the prevention and alleviation of such conditions. The compound may be administered to a subject in need of treatment by a variety of conventional routes of administration, including orally, parenterally and topically. In general, these compounds will be administered orally or parenterally at dosages between about 0.5 and 25 mg./kg. body weight of the subject to be treated per day, preferably from about 1.0 to 10 mg./kg. However, some variation in dosage will necessarily occur depending on the condition of the subject being treated. The person responsible for administration will, in any event, determine the appropriate dose for the individual subject.

The novel compound of the invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compounds of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitioneal administration. In this connection, the sterile aqueous media employed are all readily available by standard techniques known to those skilled in the art.

Compounds of formula I may be more particularly employed for the preparation of ophthalmic solutions. Such ophthalmic solutions are of principal interest for the treatment of diabetic cataracts by topical administration. For the treatment of diabetic cataracts the compounds of this invention are administered to the eye in the form of an ophthalmic preparation prepared in accordance with conventional pharmaceutical practice. The ophthalmic preparation will contain a compound of formula I or a pharmaceutically acceptable salt thereof in a concentration from about 0.01 to about 1% by weight, preferably from about 0.05 to about 0.5% in a pharmaceutically acceptable solution, suspension or ointment.

The activity of the compounds of the present invention as agents for the control of chronic diabetic complications may be determined by a number of standard biological or pharmacological tests. Suitable tests include (1) measuring their ability to inhibit the enzyme activity of isolated aldose reductase; (2) measuring their ability to reduce or inhibit sorbitol accumulation in the sciatic nerve and lens of acutely streptozotocinized, i.e. diabetic, rats; (3) measuring their ability to reverse already-elevated sorbitol levels in the sciatic nerve and lens of chronic streptozotocin-induced diabetic rats; (4) measuring their ability to prevent or inhibit galactitol formation in the lens of acutely galactosemic rats; (5) measuring their ability to delay cataract formation and reduce the severity of lens opacities in chronic galactosemic rats; (6) measuring their ability to prevent sorbitol accumulation and cataract formation in isolated rat lens inclubated with glucose; and (7) measuring their ability to reduce already elevated sorbitol levels in isolated rat lens incubated with glucose.

Proton nuclear magnetic resonance spectra ('HNMR) were measured for solutions in deuterochoroform ($CDCl_3$) and peak positions are expressed in parts per million (ppm) downfield from tetramethylsilane (TMS). The peak shapes are denoted as follows: s, singlet; d, doublet; t, triplet; q, quartet; m, multiplet; br, broad.

## EXAMPLE 1

### A. 2-Chloromethyl-5,7-dimethoxybenzothiazole

To a solution of 2-amino-5,7-dimethoxybenzothiazole, hydrochloride (2.21 g) in ethanol (20 ml) was added 2-chloro-1,1,1-triethoxyethane (1.96 g). The mixture was refluxed overnight. The solution was concentrated to remove excess ethanol and the residue purified by chromatography over silica gel using a 9:1 mixture of methylene chloride and hexane as the eluent (1.65 g; m.p. 105 °C).

### B. Ethyl-3-(5,7-Dimethoxybenzothiazole-2-ylmethyl)-4-oxo-3H-phthalazin-1-ylacetate

To a solution of ethyl 4-oxo-3H-phthalazin-1-ylacetate (1.17 g) in dimethylformamide (10 ml) was added dry powdered potassium t-butoxide (0.62 g). After stirring the solution for 15 minutes, 2-chloromethyl-5,7-dimethoxybenzothiazole (1.34 g) was added and stirred over night at room temperature. The solution was then poured onto ice-water (20 ml) containing sufficient diluted hydrochloric acid to maintain the pH at about 4. The precipitated solid was collected and air-dried to obtain the title compound (1.47 g; m.p. 154-155 °C).

### C. 3-(5,7-Dimethoxybenzothiazol-2-ylmethyl)-4-oxo-3-H-phthalazin-1-yl acetic acid

To a solution of ethyl-3-(5,7-dimethoxybenzothiazol-2-ylmethyl)-4-oxo-3-H-phthalazin-1-ylacetate (1.47 g) in 50 ml of 2:1 tetrahydrofuran-ethanol was added aqueous KOH (10% solutions, 5 ml) and stirred at room temperature for 4 hours. It was then concentrated under vacuum and acidified to pH 2 by addition of sufficient 6N HCl. The precipitated solid was collected, air-dried and crystallized from methylene chloride (100 ml) containing a few drops of methanol (1.2 g; m.p. 219-220 °C).

### D. 3-(5,7-Dihydroxy-2-benzothiazolylmethyl)-4-oxo-3-H-phthalazin-1-ylacetic acid

A mixture of 3-(5,7-dimethoxy-2-benzothiazolylmethyl)-4-oxo-3-H-phthalazin-1-ylacetic acid (0.5 g) and aqueous hydrobromic acid (10 ml, 48%) was refluxed for 4 hours. The hot solution was cooled to room temperature and then poured onto ice-water (50 ml). The resulting pink colored solid was filtered, washed with water (20 ml), dried and the dried solid was crystallized from methanol to yield the product (0.31 g; m.p. 184 °C).

## EXAMPLE 2

In accordance with Example 1D, the following compounds were prepared:

TABLE 1

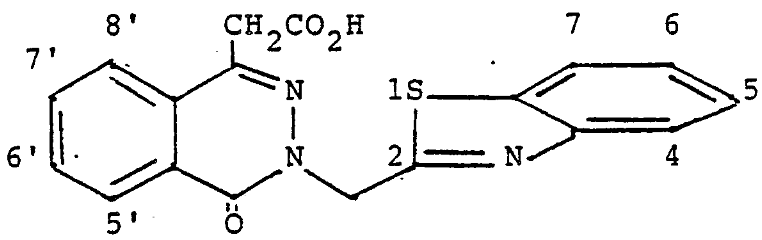

| Substituent | M.P.°C |
|---|---|
| 4-OH | 165-166 |
| 5-OH | 154-156 |

EXAMPLE 3

A. 2-Trifluoromethyl-5-methylbenzothiazole

A mixture of 2-amino-4-methyl-benzenethiol hydrochloride [10.0 g, prepared according to JACS 53, 209 (1931)], trifluoroacetic anhydride (10 ml) and methylene chloride (100 ml) was refluxed for 4 hours. The reaction mixture was concentrated under vacuum and the residue was purified by chromatography over silica gel using a 1:1 mixture of hexane and methylene chloride (6.2 g; m.p. 49-50° C).

B. 2-Trifluoromethyl-5-bromomethylbenzothiazole

A mixture of 2-trifluoromethyl-5-methylbenzothiazole (4.06 g), N-bromosuccinimide (3.92 g), carbon tetrachloride (50 ml) and a catalytic amount (0.1 g) of benzoyl peroxide was refluxed under a UV lamp for 1 hour. Upon cooling the mixture was filtered and the filtrate was evaporated to dryness. The residue was purified by chromatography over silica gel using a 95-5 mixture of methylene chloride and hexane (4.04 g), $^{1}HNMR(CDCl_3$, 60 mHz): 4.65 (s, 2H), 7.55 (m, 1H), 7-9-8-1 (m, 2H).

C. Ethyl-3-(2-trifluoromethyl-benzothiazol-5-ylmethyl)-4-oxo-3H-phthalazin-1-ylacetate

To a solution containing ethyl 4-oxo-3H-phthalazin-1-ylacetate (1.17 g) and potassium t-butoxide (0.61 g) in dimethyl formamide (10 ml) was added 2-trifluoromethyl-5-bromobenzothiazole (1.86 g). The mixture was stirred for 2 hours at room temperature and then poured onto ice-water (50 ml) containing sufficient dilute HCl to maintain the pH at about 2.0 and the precipitated solid was extracted with methylene chloride (50 ml). Upon evaporation of methylene chloride, the residue was chromatographed over silica gel (eluent, methylene chloride/ethylacete, 1:19) to obtain the title product (1.82 g; m.p. 129-130° C).

EXAMPLE 4

The following compounds of Table 2 were prepared in accordance with Example 4C using the appropriate 2-bromomethylbenzothiazoles. The melting points are in degrees Centigrade.

TABLE 2

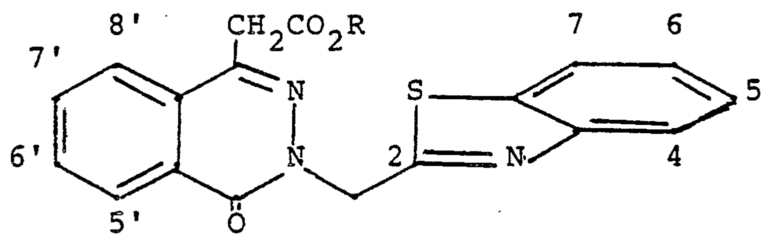

| Substituent | R | Product |
|---|---|---|
| 6-$OCH_3$ | $CH_3$ | 'HNMR($CDCl_3$,90MHz):3.70 (s,3H),3.80(s,3H),4.05(s,2H), 5.75(s,2H),7.00(dd,J=3,9Hz,1H) 7.20(d,J=3Hz,1H),7.6-7.9,(m, 4H),8.4-8.6(m,1H) |
| 5-$CF_3$, 6-$OCH_3$ | $C_2H_5$ | 'HNMR($CDCl_3$,200 MHz):1.2(t, J=8Hz,3H),3.9 (s,3H),3.96(s, 2H),4.18(9,J=8Hz,2H),7.3 (s,1H),7.85(m,3H),8.15(s,1H), and 8.4(d,J=9Hz,1H). |
| 4-$OCH_3$ | $C_2H_5$ | m.p. 149-153°C |
| 5-$OCH_3$ | $C_2H_5$ | m.p. 127-129°C |
| 5-$OCH_3$,7-$OCH_3$ | $C_2H_5$ | m.p. 154-155°C |

EXAMPLE 5

In accordance with Example 1C, the following compounds were prepared.

TABLE 3

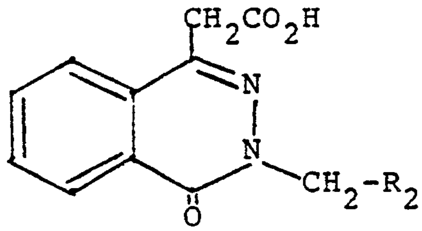

| $R_2$ | M.P.°C |
|---|---|
| 3-chlorobenzothiophen-2-yl | 186-188 |
| 3-methoxybenzothiophen-2-yl | 70-73 |
| 6-bromo-3-chlorobenzothiophen-2-yl | 211-212 |
| thieno[2,3-b]pyridine-2-yl | 201-202 |
| 7-chloro-imidazo[1,5-a]pyridine-2-yl | 219-220 |
| 2-trifluoromethylbenzothiazole-5-yl | 198 |

## EXAMPLE 6

According to Example 4C, the following compounds were prepared:

TABLE 4

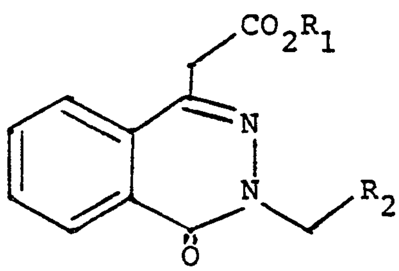

| $R_2$ | $R_1$ | Product |
|---|---|---|
| 3-chlorobenzothiophen-2-yl | $C_2H_5$ | Not characterized; used directly in the next step. |
| 3-methoxybenzothiophen-2-yl | $C_2H_5$ | $^1$HNMR ($CDCL_3$,60MHz): 1.2(t,J=8Hz,3H),3.8(s,2H),4(s,3H),4.1(q,J=8Hz,2H),7.0-7.8(m,7H),8.4-8.5(m,1H). |

| $R_2$ | $R_1$ | Product |
|---|---|---|
| 6-bromo-3-chloro-benzothiophen-2-yl | $C_2H_5$ | Not characterized; used directly in the next step. |
| thieno[2,3-b]-pyridine-2-yl | $CH_3$ | m.p. 115-122°C |
| 7-chloro-imidazo-[1,5-a]pyridine-2-yl | $CH_3$ | m.p. 188-189°C |

## EXAMPLE 7

### A. Ethyl-3-cyanomethyl-4-oxo-3H-phthalazin-1-ylacetate

To a solution of ethyl-4-oxo-3H-phthalazin-1-yl acetate (11.31 g) and dry potassium t-butoxide (5.9 g) in dimethylformamide (50 ml) was added chloroacetonitrile (3.78 g) and the solution was stirred for 30 minutes. This solution was poured onto ice water (300 ml); sufficient 10% HCL was added to adjust the pH of the resulting mixture to about 4.0 and the precipitated solid was collected and air-dried (yield: 11.81 g; m.p. 113-114 °C).

### B. Ethyl-3-(5,7-difluorobenzothiazol-2-ylmethyl)-4-oxo-3H-phthalazin-1-ylacetate

A mixture of ethyl -3-cyanomethyl-4-oxo-3H-phthalazin-1-ylacetate (1.29 g), 2-amino-4,6-difluorothiophenol hydrochloride (0.98 g) and ethanol (20 ml) was refluxed for 6 hours. Upon cooling, the title compound precipitated out as a pale yellow solid (yield: 1.62 g; m.p. 115-117 °C).

Ethyl-3-(5-methoxybenzothiazol-2-ylmethyl)-4-oxo-3H-phthalazin-1-ylacetate (m.p. 127-129 °C) was also prepared by the above procedure starting from appropriately substituted amino-thiophenol hydrochlorides.

## EXAMPLE 8

### 3-(5,7-Dihydroxy-2-benzothiazolylmethyl)-4-oxo-3H-phthalazin-lylacetic acid

A mixture of 3-(5,7-dimethoxy-2-benzothiazolylmethyl)-4-oxo-3H-phthalazin-1-yl acetic acid (0.5 g) and aqueous hydrobromic acid (10 ml, 48%) was refluxed for 4 hours. The hot solution was cooled to room temperature and than poured onto ice-water (50 ml). The resulting pink colored solid was filtered, washed with water (20 ml) and dried. The dried solid was crystallized from methanol to yield the product (0.31 g; m.p. 184 °C).

3-(4-Hydroxy-2-benzothiazolylmethyl)-4-oxo-3H-phthalazin-1-ylacetate acid (m.p. 154-156 °C) was similarly prepared starting from 3-(4-methoxy-2-benzothiazolylmethyl)-4-oxo-3H-phthalazin-1-ylacetic acid; 3-(6-hydroxy-2-benzothiazolylmethyl)-4-oxo-3H-phthalazin-1-ylacetate acid (m.p. 211 °C with dec.) from 3-(6-methoxy-2-benzothiazolylmethyl)-4-oxo-3H-phthalazin-ylacetate acid; and 3-(6-hydroxy-5-trifluoromethylbenzothiazol-2-ylmethyl-4-oxo-3H-phthalazin-1-ylacetic acid (0.15 g, m.p. 220 °C. with dec.) from ethyl-3-(6-methoxy-5-trifluoromethylbenzothiazol-2-ylmethyl)-ylacetate.

## EXAMPLE 9

3-(1,2,4-Triazolo[1,5-a]pyridin-2-ylmethyl)-4-oxo-3H-phthalazin-1-yl-acetic acid

1.5 g (4.1 mmol) 3-(s-triazolo[1,5-c]pyrimidin-2-ylmethyl)-4-oxo-3H-phthalazin-1-yl-acetic acid ethyl ester was combined with 180 mL 20% (wt - vol) potassium hydroxide in methanol and 30 mL 1,4-dioxane. After stirring at room temperature for 27 hours, the reaction was concentrated in vacuo to a solid which was partitioned between methylene chloride and water. The aqueous layer was acidified to pH 4 and the resultant white precipitate was removed by filtration, washed well with water, then with diethyl ether, and was dried at room temperature under vacuum (0.01 mm Hg) to give 0.91 g (65%) of the product: m.p. 178-181 °C.

3-(1,2,4-Triazolo[1,5-a]pyridin-2-ylmethyl)-4-oxo-3H-phthalazin-1-yl-acetic acid ethyl ester

To solution of 2.7 g (9.2 mmol) of N-aminopyridinium mesylate in 30 mL: dry dimethyl formamide at 0 °C was added 2.5 g (9.2 mmol) 3-cyanomethyl-4-oxo-3H-phthalazin-1-yl acetic acid ethyl ester and 1.3 g (9.2 mmol) anhydrous potassium carbonate. The reaction was stirred at room temperature for 70 hours and the resulting dark purple solution was concentrated in vacuo to a black residue. This was taken up in chloroform and insoluble material was removed by filtration. The chloroform solution was concentrated in vacuo and subjected to flash chromatography on silica gel using ethyl acetate as eluent to give the product as a tan solid, 1.6 g (48.5%): m.p. 98-105 °C.

EXAMPLE 10

3-(6-Chloro-1,2,4-triazolo[1,5-a]pyridin-2-ylmethyl)-4-oxo-3H-phthalazin-1-yl acetic acid

1.1 g (2.76 mmol) 3-(60-chloro-s-triazolo[1,5-a]pyrimidin-2-ylmethyl)-4-oxo-3H- phthalazin-1-yl acetic acid ethyl ester was combined with 120 mL 20% (wt -vol) potassium hydroxide in methanol and 5 mL 1,4-dioxane and was stirred for 48 hours at room temperature. The reaction was concentrated in vacuo and the residue was dissolved in water. The pH was adjusted to 3 and after 15 minutes stirring the resultant precipitate was collected by filtration, washed with water followed by diethyl ether and dried at room temperature in vacuo (0.01 mm Hg) to give 1.0 g (98%) of product: m.p. 201-203 °C.

3-(6-Chloro-1,2,4-triazolo[1,5-a]pyridin-2-ylmethyl)-4- oxo-3H phthalazin-1-yl acetic acid ethyl ester

To a slurry of 2.4 g (21.5 mmol) potassium t-butoxide in 25 mL dry dimethyl formamide at room temperature was added a slurry of 5 g (21.5 mmol) 4-oxo-3H-phthalazin-1-yl acetic acid ethyl ester in 25 mL dry dimethyl formamide. After a clear orange solution was present the reaction was cooled to 0 °C and a solution of 4.3 g (21.5 mmol) of 6-chloro-2-chloromethyl-s-triazolo[1,5-a] pyrimidine in dry dimethyl formamide at 0 °C was added dropwise. The reaction turned dark brown and was allowed to warm to room temperature and was stirred for 72 hours. The reaction was concentrated in vacuo and the residue was dissolved in chloroform. Insoluble material was removed by filtration and the chloroform solution was concentrated in vacuo. Flash chromatography on silica gel using 8-2 ethyl acetate-hexane as eluent give 1.1 g (13%) of product: m.p. 148-151 °C.

6-Chloro-2-chloromethyl-1,2,4-triazolo[1,5-a]pyridine

To a slurry of 5.36 g (30 mmol) 6-chloro-2-hydroxymethyl-s-triazolo[1,5-a]pyrimidine in 50 mL methylene chloride was added 365 mL thionylchloride. The mixture was heated at reflux for 1 hour and was cooled to room temperature and concentrated in vacuo to a brown residue. This was slurred in diethyl ether and was collected by filtration, washed with ether and dried to give 5.4 g (89%) of product. NMR($CDCl_3$):

8.60 (s, 1H); 7.71 (d, 1H); 7.55 1d of d, 1H); 4.82 (s, 2).

6-Chloro-2-hydroxymethyl-1,2,4-triazolo[1,5-a]pyridine

A mixture of 10 g (29 mmol) N-amino-2-amino-5-chloropyridine mesylate and 30 g glycolic acid was heated at 180 °C for 1 hour and then was cooled to room temperature and the pH was adjusted to 12 with 20% sodium hydroxide solution. This was extracted with methylene chloride and the organic extracts were dried over anhydrous magnesium sulfate and the filtered organic solution was concentrated in vacuo to a crude tan solid. This was taken up in methylene chloride, washed with 1N sodium hydroxide and the dried organic solution was concentrated in vacuo to a tan solid. This was repeatedly triturated with diethyl ether to give 1.62 g (30.6%) of product: m.p. 148-149 °C.

## EXAMPLE 11

The following compounds were prepared in accordance with Examples 9 or 10, as indicated.

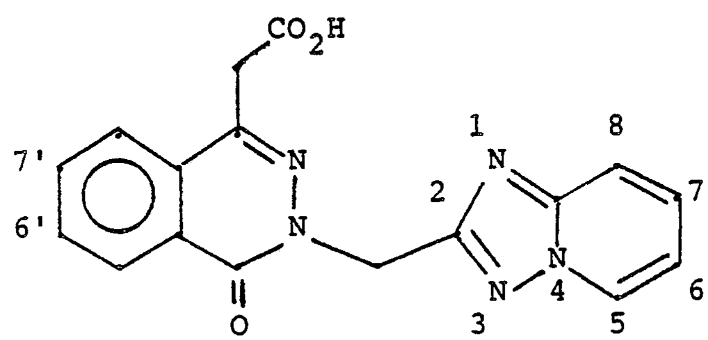

Table 5

| Substituent | Example | M.P. °C | Precursor Ethyl Ester M.P. °C |
|---|---|---|---|
| 8-Cl | 11 | 181-183 | 148-151 |
| 5-$CH_3$, 7-$CH_3$ | 11 | 199-201 | 142-144 |
| 5-$CH_3$, 6-$CH_3$ | 11 | 202-204 | 175-176 |
| 5-$CH_3$ | 11 | 198-200 | 107-108 |
| 6-$CH_3$, 8-$CH_3$ | 11 | 179-181 | 158-160 |
| 6-Cl, 8-Cl | 12 | 156-158 | 153-154 |
| 6′-Cl, 7′-Cl | 12 | 219-220 | 185-187 |
| 6-Cl, 6′-Cl, 7′-Cl | 12 | 210-213 | 234-236 |
| Substituent | Example | M.P. °C | Precursor Ethyl Ester M.P. °C |
| 7-$CH_3$, 8-$CH_3$ | 11 | 187-188 | 154-160 |
| 50$CH_3$, 8-$CH_3$ | 11 | 226 | 176-177 |
| 7-Cl | 12 | 217-218 | |
| 7-$CH_3$ | 11 | 99-102 | 129-132 |
| 6′Cl, 7′Cl, 7Cl | 12 | 203-204 | 215-220 |

## EXAMPLE A

The following intermediates were prepared according to Example 4B unless otherwise indicated.

TABLE 6

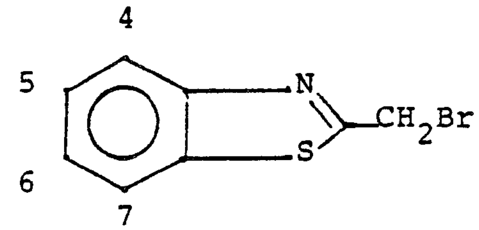

| Substituent | Product |
|---|---|
| 4-$OCH_3$ | NMR(60MHz, $CDCl_3$),3.45 (s,3H),4.85(s,2H),5.5-7.2(m,3H) |
| 5-$OCH_3$,7-$OCH_3$ | m.p. 105°C. |
| 5-$CF_3$, 6-$OCH_3$ | 'HNMR (300 MHz, $CDCl_3$): 3.91 (s, 3H), 4.74 (s,2H), 7.35 (s, 1H), and 8.13 (s, 1H) |

EXAMPLE B

2-Methyl-6-methoxy-5-trifluoromethyl benzothiazole

The title compound was prepared according to Synthesis, 1976, 730 starting from 2-bromo-4-methoxy-5-trifluoromethyl thioacetanilide (7.4 g): yield 4 g, m.p. 97-98° C.

EXAMPLE C

2-Hydroxymethyl-3-chloro-benzothiophene

2-carbomethoxy-3-chloro-benzothiophene (0.4 g), prepared according to J. Org. Chem., 21, 3037 (1975), was dissolved in ether (15 ml) and to the resulting solution was added lithium aluminium hydride (0.09 g). After 30 minutes a few drops of ethyl acetate were added to the reaction mixture. It was poured into ice-water (20 ml) and then acidified to pH 3.0 by addition of a sufficient quantity of dilute hydrochloric acid. The organic layer was separated, washed with 10-1 sodium bicarbonate solution and the organic layer was collected and dried over anhydrous magnesium sulfate. Evaporation of the dried extract gave a pale yellow solid, 'HNMR ($CDCl_3$, 60 MHz): 2.9-3.2 (b, 1H), 4.9 (s, 2H), 7.0-7.8 (m, 4H).

EXAMPLE D

2-Bromomethyl-3-chloro-benzothiophene

To an ice-cold solution of 2-hydroxymethyl-3-chloro-benzothiophene (0.35 g) in ether (15 ml) was added phosphorous tribromide (0.2 ml) and this was stirred for 1 hour. The reaction mixture was then poured onto ice-water (10 ml). The organic layer was collected, dried over anhydrous magnesium sulfate and then evaporated to dryness to obtain the title product, 'HNMR ($CDCl_3$, 60 MHz): 4.6 (s, 2H), 7.1-7.8 (m, 4H).

## EXAMPLE E

Intermediates of the formula Hal-$CH_2R_2$ were prepared by the following methods.

TABLE 7

| Structure | Product |
|---|---|
| 2-bromomethyl-3-methoxybenzothiophene | 'HNMR ($CDCl_3$,60MHz):, 4.0(s,3H),5.0(s,2H),7.2-7.8(m,4H), (Prepared according to J. Chem. Soc (C), 731 (1967)) |
| 2-chloromethyl-6-bromo-3-chlorobenzothiophene | 'HNMR ($CDCl_3$,60MHz):4.65(s,2H),7.45(s,2H),7.8(s,1H).[Prepared according to J. Med. Chem. 29, 1643 (1986)] |
| 2-chloromethylthieno[2,3-b]pyridine | m.p. 47-49° C (Prepared by the method of Example D using mesyl chloride) |
| 2-chloromethyl-7-chloroimidazo[1,2-a]pyridine | Prepared according to I1 Farmaco-Ed. Sc., 815 (1975),m.p. 122-124° C |

## EXAMPLE F

I. 2-Bromo-4-methoxy-5-trifluoromethyl aniline hydrobromide

The title compound was prepared starting from 4-methoxy-5-trifluoromethylaniline (22 g) using a standard bromination procedure with methanol as a solvent: yield, 24 g; $^1$HNMR (60 MHz, $CDCl_3$) , 3.8 (s, 3H), 6.9 (s, 1H) and 7.2 (s, 1H).

II. 2-Bromo-4-methoxy-5-trifluoromethyl acetanilide

2-Bromo-4-methoxy-5-trifluoromethyl aniline hydrobromide (18.3 g) was acetylated under standard conditions using acetic anhydride to obtain the title product: yield, 16.3 g; m.p. 155-156° C.

III. 2-Bromo-4-methoxy-5-trifluoromethyl thioacetanilide

To a solution of 2-bromo-4-methoxy-5-trifluoromethyl acetanilide (10.0 g) in benzene (100 ml) was (17.8 g) added phosphorous pentasulfide and then refluxed for 3 hours. Upon cooling the solution was filtered and the filtrate was evaporated to dryness to obtain the product: yield, 7.6 g; m.p. 130-131° C.

## EXAMPLE G

2-Amino-5,7-dimethoxybenzothiazole hydrobromide

The title compound was prepared starting from the commercially available 3.5-dimethoxyaniline and following the procedure described in U.S. Patent 4,052,379, (m.p. > 270 °C). $^1$HNMR (300 Mz, $CDCl_3$) : 3.8 (s, 3H), 7.0 (s, 1H), and 7.1 (s, 1H).

2-Amino-5,7-dimethoxybenzothiazole hydrochloride

This compound was prepared starting from 2-amino-5,7-dimethoxy-benzothiazole hydrobromide and following the procedure described in J. Chem. Soc., (c) 2148 (1969), (m.p. 211 °C).

## Claims

1. A compound of the formula

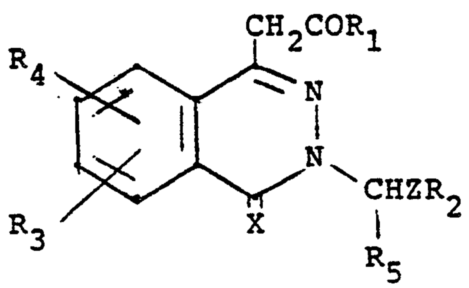

I

wherein
X is oxygen or sulfur;
Z is a covalent bond, O, S, NH or $CH_2$, or $CHR_5Z$ is vinylene;
$R_1$ is hydroxy, or a prodrug group;
$R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said heterocyclic 5-membered ring substituted by one or two phenyl, or condensed with benzo, said phenyl or benzo substituted by one of cyano, nitro, perfluoroethyl, trifluoroacetyl, or $(C_1\text{-}C_4)$alkanoyl, one or two of hydroxy or trifluoromethoxy, or one or two trifluoromethyl with one hydroxy or one $(C_1\text{-}C_4)$alkoxy, or three fluoro;
said benzo-condensed heterocyclic 5-membered ring optionally substituted in the heterocyclic 5-membered ring by one of fluoro, chlor, bromo, methoxy, or trifluoromethyl;
imidazolopyridine substituted by one of trifluoromethyl, trifluoromethylthio, bromo, or $(C_1\text{-}C_4)$alkoxy, or two of fluoro or chloro;
triazolopyridine optionally substituted by one of trifluoromethyl, trifluoromethylthio, bromo, or $(C_1\text{-}C_4)$alkoxy, or two of fluoro or chloro;
thienothiophene or thienofuran optionally substituted by one of fluoro, chloro or trifluoromethyl;
thienotriazole optionally substituted by one of chloro or trifluoromethyl;
thienopyridyl; or thienoisothiazole;
$R_3$ and $R_4$ are the same or different and are hydrogen, fluoro, chloro, bromo, trifluoromethyl, $(C_1\text{-}C_4)$alkyl, $(C_1\text{-}C_4)$alkoxy, $(C_1\text{-}C_4)$alkylthio, $(C_1\text{-}C_4)$alkylsulfinyl, $(C_1\text{-}C_4)$alkylsulfonyl, or nitro, or $R_3$ and $R_4$ taken together are $(C_1\text{-}C_4)$alkylenedioxy; and
$R_5$ is hydrogen, methyl or trifluoromethyl; or
a pharmaceutically acceptable base addition salt of a compound of formula I wherein $R_1$ is hydroxy, or an acid addition salt of a compound of formula I wherein $R_1$ is di$(C_1\text{-}C_4)$alkylamino or $(C_1\text{-}C_4)$alkoxy substituted by N-morpholino or di$(C_1\text{-}C_4)$alkylamino;
with the proviso that when $CHR_5Z$ is vinylene, $R_2$ is a heterocyclic 5-membered ring having one nitrogen, oxygen or sulfur, two nitrogens one of which may be replaced by oxygen or sulfur, or three nitrogens one of

which may be replaced by oxygen or sulfur, said heterocyclic 5-membered ring substituted by one or two fluoro, chloro, ($C_1$-$C_4$)alkyl or phenyl, or condensed with benzo, or substituted by one of pyridyl, furyl or thienyl, said phenyl or benzo optionally substituted by one of iodo, cyano, nitro, perfluoroethyl, trifluoroacetyl, or ($C_1$-$C_4$)alkanoyl, one or two of fluoro, chloro, bromo, hydroxy, ($C_1$-$C_4$)alkyl, ($C_1$-$C_4$alkoxy, ($C_1$-$C_4$)alkylthio, trifluoromethoxy, trifluoromethylthio, ($C_1$-$C_4$)alkylsulfinyl, ($C_1$-$C_4$)alkylsulfonyl, or trifluoromethyl, or two fluoro or two trifluoromethyl with one hydroxy or one ($C_1$-$C_4$)alkoxy, or three fluoro; said pyridyl, furyl or thienyl optionally substituted in the 3-position by fluoro, chloro, bromo, ($C_1$-$C_4$)alkyl or ($C_1$-$C_4$)alkoxy;

a heterocyclic 6-membered ring having one to three nitrogen atoms, or one or two nitrogen atoms and one oxygen or sulfur, said heterocyclic 6-membered ring substituted by one or two ($C_1$-$C_4$)alkyl or phenyl, or condensed with benzo, or substitued by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, ($C_1$-$C_4$)alkyl or ($C_1$-$C_4$)alkoxy;

said benzo-condensed heterocyclic 5-membered or 6-membered rings optionally substituted in the heterocyclic 5-membered or 6-membered ring by one of fluoro, chloro, bromo, ethoxy, or trifluoromethyl;

oxazole or thiazole condensed with a 6-membered aromatic group containing one or two nitrogen atoms, with thiophene or with furane, each optionally substituted by one of fluoro, chloro, bromo, trifluoromethyl, methylthio or methylsulfinyl;

imidazolopyridine or triazolopyridine optionally substituted by one of trifluoromethyl, trifluoromethylthio, bromo, or ($C_1$-$C_4$)alkoxy, or two of fluoro or chloro;

thienothiophene or thienofuran optionally substituted by one of fluoro, chloro or trifluoromethyl;

thienotriazole optionally substituted by one of chloro or trifluoromethyl;

naphthothiazole, naphthoxazole; thienopyridyl; or thienoisothiazole.

2. A compound according to claim 1 characterized in that X is oxygen, Z is a covalent bond, $R_1$ is hydroxy, and $R_3$, $R_4$ and $R_5$ are each hydrogen.

3. A compound according to claim 1 or 2, characterized in that $R_2$ is substituted benzothiazolyl, benzoxazolyl, oxadiazolyl, isoquinolyl, benzothiophenyl, benzofuranyl, benzimidazolyl, indolyl, or benzo-substituted benzothiazolyl, benzoxazolyl, benzimidazolyl, benzothiophenyl, benzofuranyl, or indolyl.

4. A compound according to claim 1 or 2, characterized in that $R_2$ is 2-benzothiazolyl substituted in the benzo by one or two of hydroxy, or trifluoromethyl, or two fluoro or two trifluoromethyl with one methoxy, or three fluoro

5. A compound according to any one of claims 1 to 3, characterized in that $R_3$ is hydrogen, 5-fluoro, 5-chloro, 5-bromo or 5-methyl, and $R_4$ is hydrogen, 6-or 7- substituted chloro, bromo, methyl, isopropyl, methoxy, nitro or trifluoromethyl; or $R_3$ and $R_4$ is 4,5-difluoro.

6. A compound according to claim 1 or 2 wherein $R_2$ is benzothiazol-2-yl substituted by one hydroxy and one trifluoromethyl, or triazolopyridinyl.

7. A compound according to claim 1 herein $CHR_5Z$ is vinylene and $R_2$ is optionally substituted benzothiazolyl.

8. A compound according to claim 7 wherein $R_2$ is benzothiazolyl substituted by one or two of fluoro, chloro, bromo or trifluoromethyl.

9. A pharmaceutical composition characterized by comprising a compound of claim 1 in admixture with a pharmaceutically acceptable carrier.

10. A compound according to any one of claims 1 to 8 for use as a medicament.

Claims for the following Contracting States: ES, GR

1. A process for preparing a compound of the formula

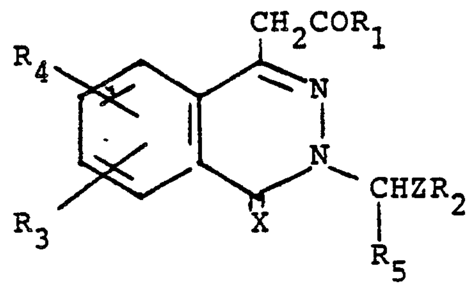

I

wherein
X is oxygen or sulfur;
Z is a covalent bond, O, S, NH or $CH_2$, or $CHR_5Z$ is vinylene;
$R_1$ is hydroxy, or a prodrug group;
$R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said heterocyclic 5-membered ring substituted by one or two phenyl, or condensed with benzo, said phenyl or benzo substituted by one of cyano, nitro, perfluoroethyl, trifluoroacetyl, or ($C_1$-$C_4$)alkanoyl, one or two of hydroxy or trifluoromethoxy, or one or two trifluoromethyl with one hydroxy or one ($C_1$-$C_4$)alkoxy, or three fluoro;
said benzo-condensed heterocyclic 5-membered ring optionally substituted in the heterocyclic 5-membered ring by one of fluoro, chloro, bromo, methoxy, or trifluoromethyl;
imidazolopyridine substituted by one of trifluoromethyl, trifluoromethylthio, bromo, or ($C_1$-$C_4$)alkoxy, or two of fluoro or chloro;
triazolopyridine optionally substituted by one of trifluoromethyl, trifluoromethylthio, bromo, or ($C_1$-$C_4$)alkoxy, or two of fluoro or chloro;
thienothiophene or thienofuran optionally substituted by one of fluoro, chloro or trifluoromethyl;
thienotriazole optionally substituted by one of chloro or trifluoromethyl;
thienopyridyl; or thienoisothiazole;
$R_3$ and $R_4$ are the same or different and are hydrogen, fluoro, chloro, bromo, trifluoromethyl, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or nitro, or $R_3$ and $R_4$ taken together are ($C_1$-$C_4$)alkylenedioxy; and
$R_5$ is hydrogen, methyl or trifluoromethyl; or
a pharmaceutically acceptable base addition salt of a compound of formula I wherein $R_1$ is hydroxy, or an acid addition salt of a compound of formula I wherein $R_1$ is di($C_1$-$C_4$)alkylamino or ($C_1$-$C_4$)alkoxy substituted by N-morpholino or di($C_1$-$C_4$)alkylamino;
with the proviso that when $CHR_5Z$ is vinylene, $R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said heterocyclic 5-membered ring substituted by one or two fluoro, chloro, ($C_1$-$C_4$)alkyl or phenyl, or condensed with benzo, or substituted by one of pyridyl, furyl or thienyl, said phenyl or benzo optionally substituted by one of iodo, cyano, nitro, perfluoroethyl, trifluoroacetyl, or ($C_1$-$C_4$)alkanoyl, one or two of fluoro, chloro, bromo, hydroxy, ($C_1$-$C_4$)alkyl, ($C_1$-$C_4$alkoxy, ($C_1$-$C_4$)alkylthio, trifluoromethoxy, trifluoromethylthio, ($C_1$-$C_4$)alkylsulfinyl, ($C_1$-$C_4$)alkylsulfonyl, or trifluoromethyl, or two fluoro or two trifluoromethyl with one hydroxy or one ($C_1$-$C_4$)alkoxy, or three fluoro;
said pyridyl, furyl or thienyl optionally substituted in the 3-position by fluoro, chloro, bromo, ($C_1$-$C_4$)alkyl or ($C_1$-$C_4$)alkoxy;
a heterocyclic 6-membered ring having one to three nitrogen atoms, or one or two nitrogen atoms and one oxygen or sulfur, said heterocyclic 6-membered ring substituted by one or two ($C_1$-$C_4$)alkyl or phenyl, or condensed with benzo, or substitued by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, ($C_1$-$C_4$)alkyl or ($C_1$-$C_4$)alkoxy;
said benzo-condensed heterocyclic 5-membered or 6-membered rings optionally substituted in the heterocyclic 5-membered or 6-membered ring by one of fluoro, chloro, bromo, methoxy, or trifluoromethyl;
oxazole or thiazole condensed with a 6-membered aromatic group containing one or two nitrogen atoms, with thiophene or with furane, each optionally substituted by one of fluoro, chloro, bromo, trifluoromethyl, methylthio or methylsulfinyl;
imidazolopyridine or triazolopyridine optionally substituted by one of trifluoromethyl, trifluoromethylthio,

bromo, or ($C_1$-$C_4$)alkoxy, or two of fluoro or chloro;
thienothiophene or thienofuran optionally substituted by one of fluoro, chloro or trifluoromethyl;
thienotriazole optionally substituted by one of chloro or trifluoromethyl;
naphthothiazole, naphthoxazole; thienopyridyl; or thienoisothiazole, characterized by reacting a compound of the formula

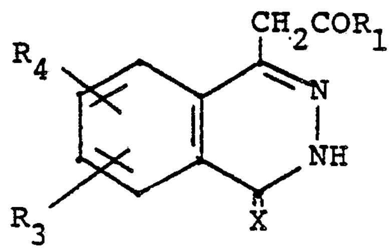

IV

with a compound of the formula

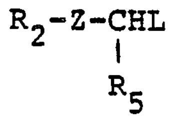

in an inert atmosphere, wherein $R_1$ is ($C_1$-$C_4$)alkoxy, X, Z, $R_2$, $R_3$, $R_4$ and $R_5$ are as defined above, and L is a leaving group, or reacting a compound of the formula

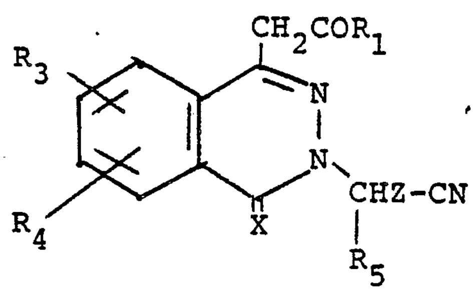

V

wherein X, $R_3$, $R_4$, $R_5$ and Z are as defined above, and $R_1$ is ($C_1$-$C_4$)alkoxy, with a compound of the formula

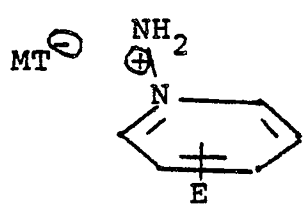

wherein E is hydrogen, one of trifluoromethyl, trifluoromethylthio, bromo, or ($C_1$-$C_4$)alkoxy, or two of fluoro or chloro, and MT is mesylate or tosylate, to form corresponding compounds of formula I wherein $R_2$ is triazolopyridine optionally substituted by E as defined above, and
when said heterocyclic 5-membered ring is substituted in said phenyl or benzo by one or two hydroxy, or one or two trifluoromethyl with one hydroxy, hydrolyzing the corresponding methoxy derivative of formula I.

2. A process according to claim 1 characterized in that X is oxygen, Z is a covalent bond, $R_1$ is hydroxy, and $R_3$, $R_4$ and $R_5$ are each hydrogen.

3. A process according to claim 1 or 2, characterized in that $R_2$ is substituted benzothiazolyl, benzoxazolyl, oxadiazolyl, isoquinolyl, benzothiophenyl, benzofuranyl, benzimidazolyl, indolyl, or benzo-substituted benzothiazolyl, benzoxazolyl, benzimidazolyl, benzothiophenyl, benzofuranyl, or indolyl.

4. A process according to claim 1 or 2, characterized in that $R_2$ is 2-benzothiazolyl substituted in the benzo by one or two of hydroxy, or trifluoromethyl, or two fluoro or two trifluoromethyl with one methoxy, or three fluoro

5. A process according to any one of claims 1 to 3, characterized in that $R_3$ is hydrogen, 5-fluoro, 5-chloro, 5-bromo or 5-methyl, and $R_4$ is hydrogen, 6-or 7- substituted chloro, bromo, methyl, isopropyl, methoxy, nitro or trifluoromethyl; or $R_3$ and $R_4$ is 4,5-difluoro.